# FASCICULE DE BREVET EUROPEEN

(11) **EP 1 180 052 B1**
(45) Date de publication et mention de la délivrance du brevet: **05.07.2006**
(21) Numéro de dépôt: 00929615.3
(22) Date de dépôt: 12.05.2000
(51) Int. Cl.: A61M 35/00, B65D 47/42

(54) **APPLICATEUR CUTANE A CARTOUCHE DE STOCKAGE DE LIQUIDE**
KARTUSCHE ZUM AUFBEWAHREN UND AUFTRAGEN EINER FLÜSSIGKEIT AUF DIE HAUT
CUTANEOUS APPLICATOR WITH A LIQUID STORAGE CARTRIDGE

(30) Priorité: 21.05.1999 FR 9906519
(43) Date de publication de la demande: 20.02.2002
(73) Titulaire: EMERIT, Michel, 95110 Sannois (FR); PATERNOTTE, Yanick, 95110 Sannois (FR)
(72) Inventeur: EMERIT, Michel, 95110 Sannois (FR); PATERNOTTE, Yanick, 95110 Sannois (FR)
(74) Mandataire: Blot, Philippe Robert Emile
(86) Numéro de dépôt international: PCT/FR2000/001298
(87) Numéro de publication internationale: WO 2000/071198

(56) Documents cités:
- EP-A- 0 320 131
- WO-A-96/40445
- FR-A- 2 770 410
- US-A- 4 913 682

## Description

La présente invention concerne un applicateur cutané de liquide du type décrit dans le préambule de la revendication 1.

Il est courant d'utiliser un tampon hydrophile pour appliquer sur une blessure un liquide désinfectant ou une substance médicamenteuse.

Afin d'éviter un dessèchement trop rapide du tampon hydrophile, durant son stockage, il est connu de stocker séparément le tampon hydrophile et le liquide destiné à imprégner le tampon.

Par exemple, le document WO-91112197 décrit un dispositif d'application cutanée comportant une cartouche de stockage sur laquelle est rapportée une tête d'application du liquide. La tête d'application comporte une structure porteuse sur laquelle est monté un tampon hydrophile. La tête d'application est fabriquée à l'écart de la cartouche puis montée sur celle-ci, par des moyens d'accouplement mécaniques tels que des moyens d'encliquetage. Afin de permettre l'ouverture de la cartouche, au moins une partie de la structure porteuse est montée déplaçable.

De ce fait, la tête d'application est de structure relativement complexe, augmentant considérablement le coût de l'applicateur cutané.

De même, FR-A-2 770 410 décrit un applicateur cutané conforme au préambule de la revendication 1, dont la structure est complexe.

L'invention a pour but de proposer un applicateur cutané dont le coût de fabrication est réduit, tout en permettant que le liquide à appliquer soit initialement confiné dans une cartouche de stockage.

A cet effet, l'invention a pour objet un applicateur cutané de liquide du type précité, caractérisé par la partie caractérisante de la revendication 1.

Des modes particuliers de réalisation sont décrits dans les revendications 2 à 7.

L'invention sera mieux comprise à la lecture de la description qui va suivre, donnée uniquement à titre d'exemple et faite en se référant aux dessins sur lesquels :
Les figures 1 et 2 sont des vues en élévation, respectivement de face et de côté, d'un applicateur cutané selon l'invention;
La figure 3 est une vue en élévation de face de l'applicateur cutané des figures 1 et 2, représenté lors de l'ouverture de la cartouche ;
Les figures 4 et 5 sont des vues en élévation de variantes de réalisation d'un applicateur cutané selon l'invention;
Les figures 6 et 7 sont des vues en élévation d'un même mode de réalisation d'un applicateur cutané selon l'invention représenté respectivement avant ouverture et lors de l'ouverture de la sortie d'écoulement du liquide ; et
La figure 8 est une vue en élévation d'encore un mode de réalisation d'un applicateur cutané selon l'invention.

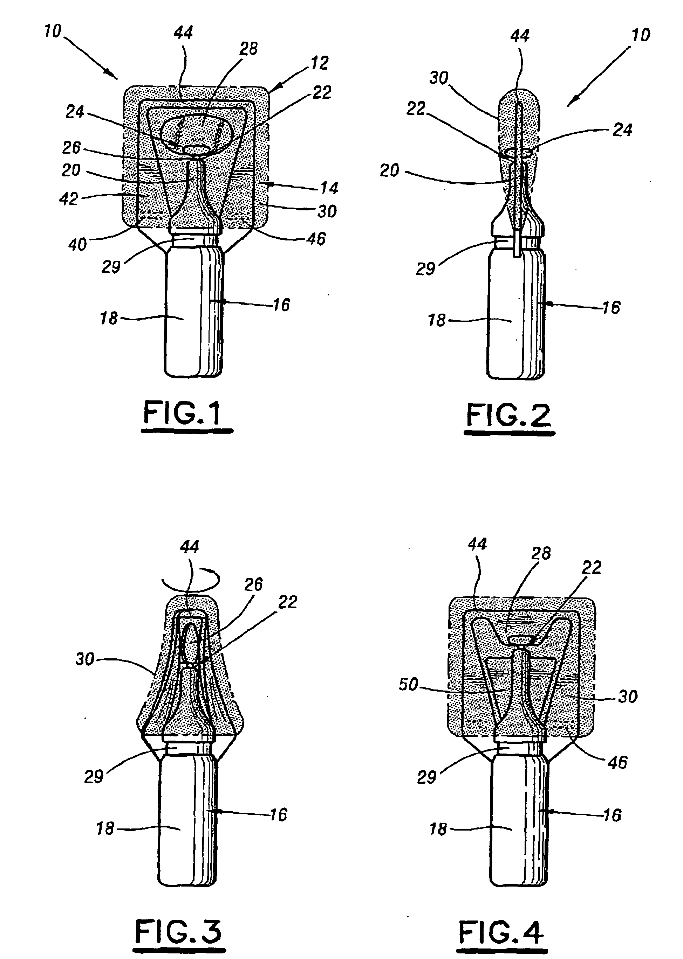

Sur les figures 1 à 3, est représenté un applicateur cutané 10 comportant un tampon hydrophile 12 retenu par des moyens de maintien 14 sur une cartouche 16 de stockage d'un liquide actif aseptique. Ce liquide est par exemple un désinfectant ou une substance médicamenteuse.

La cartouche 16 est réalisée en matière plastique moulée. Elle est réalisée par pressage entre deux demi-toutes d'un tube de polymère obtenu en sortie d'une extrudeuse. Lors du pressage, le tube de polymère est maintenu sous pression par un soufrage d'air intérieur.

La cartouche comporte un corps flexible sensiblement cylindrique 18. Le corps est prolongé à sa partie supérieure par un col ou goulot 20 de section extérieure progressivement décroissante vers son extrémité libre. Le goulot 20 délimite intérieurement un conduit d'acheminement du liquide jusqu'à une sortie axiale 22. Initialement, la sortie 22 est obturée par un bouchon sécable 24 venu de matière avec le goulot 20.

Le bouchon 24 est relié à l'extrémité du goulot pour obturer la sortie 22 par un téton frangible 26 permettant la désolidarisation du bouchon 24 et l'ouverture de la cartouche, par déplacement angulaire du bouchon par rapport au corps de la cartouche.

A cet effet, le bouchon 24 est solidaire d'une saillie d'actionnement 28 constituée par exemple par une languette en forme d'ellipse venue de matière avec le bouchon.

A la base du goulot 20, le corps 18 de la cartouche présente une gorge périphérique 29.

La contenance de la cartouche est adaptée pour renfermer la quantité nécessaire de liquide pour un usage unique. Cette contenance est comprise entre 1 ml et 5 ml et est par exemple égale à 2,5 ml, sans que ces valeurs soient considérées comme limitatives.

Dans l'applicateur 10 représenté sur les figures 1 à 3, un tampon hydrophile 12 est retenu par les moyens 14 de maintien sur la cartouche de stockage en regard de la sortie 22 d'écoulement du liquide hors de celle-ci. Le tampon 12 est formé d'une bande hydrophile 30, par exemple de la gaze.

La bande hydrophile 30 est repliée autour des moyens de maintien 14, de sorte que la sortie d'écoulement 22 de la cartouche est enchâssée entre les deux bords rabattus de la bande hydrophile 30.

A cet effet, les moyens de maintien 14 comportent un arceau 40 venu de matière à ses extrémités avec la cartouche 16. L'arceau 40, présentant une forme générale de U, surmonte la sortie d'écoulement 22 et le bouchon 24.

L'arceau 40 comporte deux jambes latérales 42 reliées l'une à l'autre par un pontet transversal 44 surmontant la sortie d'écoulement 22. L'arceau 40 délimite une arche à l'intérieur de laquelle s'étendent initialement le goulot 20, le bouchon 24 et la languette 28. Le bouchon 24 et la languette 28 sont séparés de l'arceau 40 par un intervalle périphérique.

Les jambes 42 sont reliées, à une de leurs extrémités, latéralement de part et d'autre du corps 18 dans la région supérieure de celui-ci où est ménagée la gorge 29. Les jambes 42 s'étendent dans le plan longitudinal médian de la cartouche. Les deux jambes 42 et le pontet 44 sont venus de matière avec la cartouche.

Pour la réalisation de l'arceau 40, les deux demi-moules adaptés pour la fabrication de la cartouche 16 comportent, dans la partie supérieure de l'empreinte délimitant la cartouche, des canaux en forme générale de U délimitant la forme de l'arceau 40. Ainsi, lorsque les deux demi-moules sont en contact, les deux parois opposées du tube de polymère sont comprimées l'une contre l'autre dans les canaux en forme de U formant ainsi l'arceau 40.

En outre, lors du rapprochement des deux demi-moules, ceux-ci ne sont pas appliqués parfaitement en contact l'un de l'autre, de sorte qu'un voile est formé tout autour de la cartouche 18 et de l'arceau 40, ainsi qu'à l'intérieur de la zone délimitée par l'arceau 40.

Ce voile est supprimé, après démoulage de la cartouche, par découpe de celui-ci suivant le contour extérieur de la cartouche 18 et de l'arceau 40, ainsi que par découpe du voile à l'intérieur de la zone délimitée par l'arceau 40, le long de la périphérie intérieure de celui-ci. Cette découpe s'effectue avantageusement par un outil de poinçonnage.

Comme représenté la largeur des jambes 42 décroît progressivement depuis le corps 18 de la cartouche jusqu'au pontet transversal 44. Le pontet 44 constitue un support de l'extrémité recourbée en épingle à cheveu de la bande hydrophile 30.

En outre, à la base des jambes 42, c'est-à-dire à leur extrémité de liaison avec la cartouche ces picots 46 de solidarisation de la bande hydrophile 30 sont prévus sur les deux faces opposées des jambes. Ces picots sont adaptés pour pénétrer au travers de la structure poreuse de la bande hydrophile afin d'y être fondus et ainsi d'assurer la soudure des extrémités rabattues de la bande sur les moyens de maintien 14.

Comme illustré sur la figure 3 les jambes 42 ont des dimensions permettant leur déformation élastique suivant un profil d'hélice afin d'autoriser un débattement angulaire du pontet 44 lors de l'entraînement manuel de celui-ci.

Ainsi, l'arceau peut être déformé par torsion autour de l'axe longitudinal de la cartouche.

Les moyens de maintien 14 sont ainsi déformables élastiquement entre une position de repos représentée sur la figure 2, dans laquelle l'arceau est plan, et une position de sectionnement du bouchon 24, représentée sur la figure 3, dans laquelle les jambes 42 de l'arceau sont déformées sous forme d'hélice, alors que le bouchon 24 est détaché par cisaillement.

En effet, on comprend que le corps de la cartouche étant maintenu entre les doigts, un mouvement de rotation du tampon hydrophile 12 autour de l'axe de la cartouche 16, sous l'action des doigts sollicitant l'extrémité libre du tampon, provoque le cisaillement du bouchon 24 et ainsi l'ouverture de la cartouche. Lors de la déformation par torsion du tampon hydrophile et de l'arceau, la languette 28 est simultanément sollicitée, ce qui conduit à la rupture du téton 26 liant le bouchon 24 au goulot.

Après désolidarisation du bouchon, le liquide contenu dans la cartouche est libre de s'écouler pour imbiber la bande hydrophile, notamment sous l'action d'une pression manuelle exercée sur le corps flexible de la cartouche.

Le tampon étant ainsi imprégné, l'utilisateur peut badigeonner la zone à traiter avec le tampon tout en ne maintenant que le corps de la cartouche.

On conçoit que les moyens de maintien 14 de la bande hydrophile étant venus de matière avec la cartouche, ceux-ci ont un coût de fabrication très faible, puisqu'ils sont réalisés simultanément à la fabrication de la cartouche.

Sur les figures suivantes, sont représentées des variantes de réalisation de l'applicateur selon l'invention.

Sur ces figures, les éléments identiques ou analogues à ceux des figures 1 à 3 sont désignés par les mêmes numéros de référence.

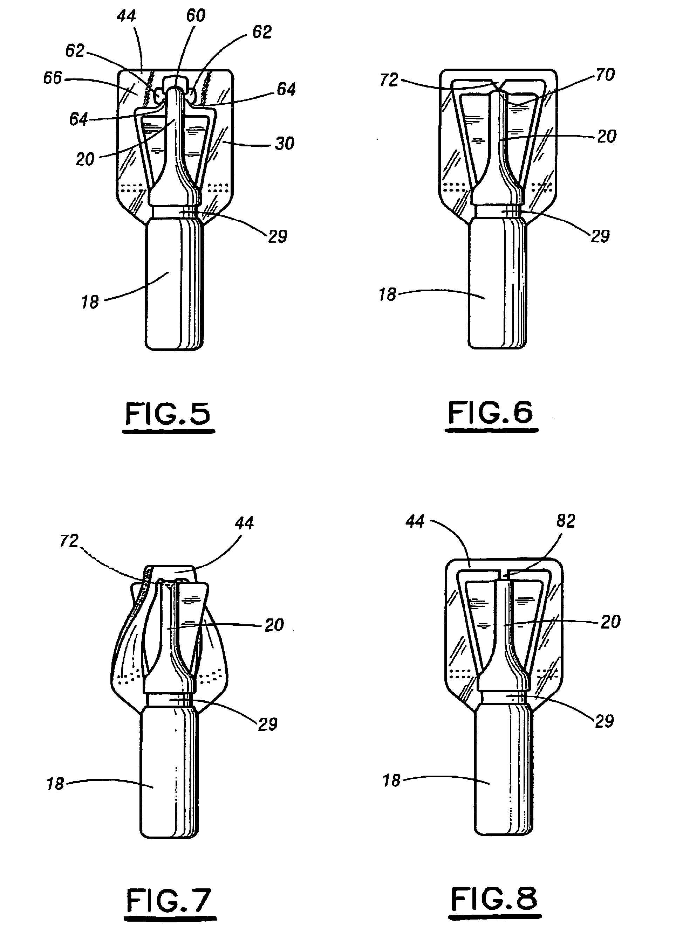

Dans le mode de réalisation de la figure 4, la languette 28 d'actionnement du bouchon 24 est venue de matière avec le pontet 44. Ainsi, lors de l'entraînement en rotation de l'arceau 40, le bouchon 24 se trouve cisaillé, même si l'opérateur n'applique ses doigts que sur le pontet 44. En outre, dans ce mode de réalisation, le goulot 20 comporte longitudinalement des nervures de renfort 50 disposées longitudinalement de part et d'autre dans le plan de l'arceau 40.

Pour des raisons de clarté des dessins, sur les figures 5 et suivantes, la bande hydrophile 30 n'est pas représentée.

Dans le mode de réalisation de la figure 5, le goulot 20 est obturé axialement par une paroi d'extrémité 60 non sécable. En revanche, le goulot est pourvu, à son extrémité libre, de deux sorties d'écoulement latérales diamétralement opposées et disposées dans le plan de l'arceau 40. Ces sorties d'écoulement sont obturées par des bouchons 62 venus de matière avec le goulot et reliés à celui-ci par des tétons frangibles 64 s'étendant radialement par rapport à l'axe du goulot.

Les bouchons 62 sont liés au pontet 44 de l'arceau par des prolongements 66 prévus dans l'angle délimité entre les jambes 42 et le pontet 44.

Dans ce mode de réalisation également, on comprend que, lors du déplacement angulaire du pontet 44 par rapport à la cartouche 16 autour de l'axe de cette dernière, les bouchons 62 sont entraînés, provoquant la rupture des tétons frangibles 64, et ainsi l'ouverture des sorties d'écoulement du liquide.

Dans les modes de réalisation des figures 6 à 8, le goulot 20 de la cartouche est obturé axialement par un opercule perforable 70 formant un bouchon libérable. En regard de l'opercule perforable 70 est disposé initialement un organe de perforation 72 porté par le pontet 44. Cet organe de perforation est avantageusement venu de matière avec le pontet 44 et est constitué d'une pointe s'étendant suivant l'àxe du goulot 20. L'extrémité pointue de l'organe de perforation 72 est initialement disposée au voisinage immédiat de l'opercule 70.

Dans le mode de réalisation des figures 6 et 7, les jambes 42 de l'arceau sont déformables élastiquement sous forme d'hélice comme dans les modes de réalisation précédents: Ainsi, ann de procéder à l'ouverture de la cartouche, et comme illustré sur la figure 7, l'opérateur déplace angulairement le pontet 44 par rapport à la cartouche provoquant ainsi la déformation des jambes.

Lors de cette déformation, la pointe 72 subit, d'une part, un mouvement de rotation autour de l'axe de la cartouche, et, d'autre part, un déplacement axial en direction du goulot 20, du fait de la déformation des jambes 42 de longueur constante sous forme d'hélice.

Ainsi, la pointe 72 provoque la perforation de l'opercule 70 et l'ouverture de la cartouche.

Dans le mode de réalisation représenté sur la figure 8, le pontet 44 est lié à l'extrémité du goulot 20 par un téton 82 formant bouchon et assurant l'obturation du conduit d'écoulement délimité par le goulot 20. Le téton 82 est venu de matière avec le goulot 20 et le téton 44.

Dans ce mode de réalisation, l'ouverture de la cartouche s'effectue par rotation du pontet 44 autour de l'axe de la cartouche. Cette rotation provoque la rupture de la liaison entre le téton 82 et l'extrémité du goulot 20, assurant ainsi l'ouverture de la cartouche 16.

De plus, l'ouverture peut également être obtenue par pression axiale sur le pontet 44 pour enfoncer temporairement le téton 82 dans le goulot 20, assurant ainsi l'ouverture de la cartouche.

## Revendications

1. Applicateur cutané de liquide (10), comportant une cartouche (16) de stockage du liquide initialement hermétiquement scellée et munie d'une sortie (22) d'écoulement du liquide obturée par un bouchon libérable (24, 62, 70) qui est sécable sous l'action d'un déplacement angulaire par rapport à l'axe de la cartouche, un tampon hydrophile (12) d'application de liquide sur une zone à traiter, et des moyens (14) de maintien du tampon hydrophile (12) en regard d'une sortie d'écoulement du liquide hors de la cartouche (16), lesdits moyens (14) de maintien comportant un arceau (40) de support du tampon (12) surmontant la sortie (22) d'écoulement du liquide hors de la cartouche, l'arceau (40) comportant des jambes latérales (42), **caractérisé en ce que** les moyens (14) de maintien du tampon sont venus de matière avec la cartouche (16) et déformables élastiquement entre une position de repos et une position de libération du bouchon (24, 62, 70), l'arceau est venu de matière à ses extrémités avec la cartouche (16), et les jambes latérales (42) sont reliées, à leurs extrémités, à la cartouche (16), les jambes étant déformables élastiquement sous forme d'hélice pour permettre une torsion de l'arceau (40).

2. Applicateur selon la revendication 1, **caractérisé en ce que** la cartouche (16) comporte un corps (18) de stockage du liquide, prolongé par un goulot (20) de section réduite, délimitant un conduit d'acheminement du liquide jusqu'à la sortie d'écoulement (22), et **en ce que** l'arceau (30) est relié à la cartouche à la base du goulot (20).

3. Applicateur selon la revendication 2, **caractérisé en ce que** le goulot (20) comporte au moins une nervure longitudinale de renfort (50).

4. Applicateur selon l'une quelconque des revendications 1 à 3, **caractérisé en ce que** le bouchon (24) est séparé de l'arceau et comporte au moins une saillie d'actionnement (28).

5. Applicateur selon l'une quelconque des revendications 1 à 3, **caractérisé en ce que** la bouchon (24 ; 62) est solidaire dudit arceau (40) pour son déplacement angulaire par rapport au corps lors de la déformation élastique de l'arceau (40).

6. Applicateur selon l'une des revendications 1 à 3, **caractérisé en ce qu'**il comporte un organe de perforation (72) du bouchon d'obturation (70), cet organe étant porté par ledit arceau (40) et disposé en regard de ladite ouverture (22).

7. Applicateur selon l'une quelconque des revendications précédentes, **caractérisé en ce que** ledit tampon hydrophile (12) comporte une bande hydrophile (30) repliée sur elle-même et les moyens de maintien (14) sont adaptés pour le maintien de la sortie (22) d'écoulement de la cartouche enchâssée entre les bords rabattus de la bande (30).

## Claims

1. Cutaneous applicator for liquid (10), comprising a cartridge (16) for storage of the liquid which is initially hermetically sealed and is provided with an outlet (22) for flow of the liquid which is closed by a stopper (24, 62, 70) which can be released, and can be separated under the action of angular displacement relative to the axis of the cartridge, an absorbent pad (12) for application of liquid onto an area to be treated, and means (14) for retention of the absorbent pad (12) opposite an outlet for flow of the liquid out of the cartridge (16), the said retention means (14) comprising an arch (40) to support the pad (12) which surmounts the outlet (22) for flow of the liquid out of the cartridge, the arch (40) comprising lateral legs (42), **characterised in that** the means (14) for retention of the pad are integral with the cartridge (16) and are deformable resiliently between a position of rest and a position of release of the stopper (24, 62, 70), the arch is integral at its ends with the cartridge (16), and the lateral legs (42) are connected at their ends to the cartridge (16), the legs being deformable resiliently in the form of a helix in order to permit twisting of the arch (40).

2. Applicator according to claim 1, **characterised in that** the cartridge (16) comprises a body (18) for storage of the liquid which is extended by a neck (20) with a reduced cross-section, which delimits a duct for conveying liquid to the flow outlet (22), and **in that** the arch (30) is connected to the cartridge at the base of the neck (20).

3. Applicator according to claim 2, **characterised in that** the neck (20) comprises at least one longitudinal reinforcement rib (50).

4. Applicator according to any one of claims 1 to 3, **characterised in that** the stopper (24) is separated from the arch and comprises at least one actuating projection (28) .

5. Applicator according to any one of claims 1 to 3, **characterised in that** the stopper (24; 62) is integral with the said arch (40) for its angular displacement relative to the body during resilient deformation of the arch (40).

6. Applicator according to any one of claims 1 to 3, **characterised in that** it comprises a unit (72) for perforation of the closure stopper (70), this unit being supported by the said arch (40) and disposed opposite the said opening (22).

7. Applicator according to any one of the preceding claims, **characterised in that** the said absorbent pad (12) comprises an absorbent strip (30) which is folded back upon itself and the means for retention (14) are designed for retention of the flow outlet (22) of the cartridge which is embedded between the folded back edges of the strip (30).

## Patentansprüche

1. Applikator zum Auftragen von Flüssigkeit (10) auf die Haut, mit einer Kartusche (16) zum Aufbewahren der Flüssigkeit, die zunächst hermetisch versiegelt ist, ausgerüstet mit einem Auslaß (22) für den Austritt der Flüssigkeit, die durch einen lösbaren Stopfen (24, 62, 70) verschlossen ist, der unter der Wirkung einer Verdrehung in Bezug auf die Achse der Kartusche abtrennbar ist, einem hydrophilen Tampon (12) zum Auftragen der Flüssigkeit auf eine zu behandelnde Zone, und Mitteln (14) zum Halten des hydrophilen Tampons (12) vor einem Auslaß für den Austritt der Flüssigkeit aus der Kartusche (16), wobei die genannten Haltemittel (14) einen zur Abstützung des Tampons (12) dienenden Bügel (40) aufweisen, der den Auslaß (22) für den Austritt der Flüssigkeit aus der Kartusche überwölbt, wobei der Bügel (40) seitliche Schenkel (42) aufweist, **dadurch gekennzeichnet, daß** die Haltemittel (14) für den Tampon in einem Stück mit der Kartusche (16) ausgebildet und elastisch zwischen einer Ruhestellung und einer Freigabestellung für den Stopfen (24, 62, 70) verformbar sind, wobei der Bügel an seinen Enden in einem Stück mit der Kartusche (16) verbunden ist und die seitlichen Schenkel (42) an ihren Enden mit der Kartusche (16) verbunden sind, wobei die Schenkel elastisch zu einer Helix verformbar sind, um eine Torsion des Bügels (40) zu ermöglichen.

2. Applikator nach Anspruch 1, **dadurch gekennzeichnet; daß** die Kartusche (16) einen Körper (18) zur Speicherung der Flüssigkeit aufweist, der durch einen Hals (20) mit reduziertem Querschnitt verlängert ist, der eine Leitung zu Zuführen der Flüssigkeit bis zu der Auslaßöffnung (22) begrenzt, und daß der Bügel (30) an der Basis des Halses (20) mit der Kartusche verbunden ist.

3. Applikator nach Anspruch 2, **dadurch gekennzeichnet, daß** der Hals (20) wenigstens eine längsverlaufende Verstärkungsrippe (50) aufweist.

4. Applikator nach einem der Ansprüche 1 bis 3, **dadurch gekennzeichnet, daß** der Stopfen (24) von dem Bügel getrennt ist und wenigstens einen Betätigungsvorsprung (28) aufweist.

5. Applikator nach einem der Ansprüche 1 bis 3, **dadurch gekennzeichnet, daß** der Stopfen (24; 62) mit dem Bügel (40) verbunden ist, so daß er bei der elastischen Verformung des Bügels (40) in Bezug auf den Körper verdreht wird.

6. Applikator nach einem der Ansprüche 1 bis 3, **dadurch gekennzeichnet, daß** er ein Organ (72) zur Perforation des Verschlußstopfens (70) aufweist und daß die dieses Organ an dem Bügel (40) gehalten und gegenüberliegend zu der genannten Öffnung (22) angeordnet ist.

7. Applikator nach einem der vorstehenden Ansprüche, **dadurch gekennzeichnet, daß** der hydrophile Tampon (12) ein auf sich selbst zurückgefaltetes hydrophiles Band (30) aufweist und daß die Haltemittel (14) dazu eingerichtet sind, die Auslaßöffnung (22) der Kartusche zwischen den umgefalteten Rändern des Bandes (30) eingeschlossen zu halten.
